# EUROPÄISCHE PATENTANMELDUNG

(11) **EP 2 226 097 A2**
(43) Veröffentlichungstag der Anmeldung: **08.09.2010**
(21) Anmeldenummer: 10154683.6
(22) Anmeldetag: 25.02.2010
(51) Int. Cl.: A61N 2/06

(54) **Vorrichtung zur lokalen Beeinflussung des Erdmagnetfeldes**

(30) Priorität: 05.03.2009 AT 3632009
(71) Anmelder: Wiebecke, Adolf, 5411 OBERALM (AT)
(72) Erfinder: Wiebecke, Adolf, 5411 OBERALM (AT)
(74) Vertreter: Babeluk, Michael

(57) **Zusammenfassung**

Die Erfindung betrifft eine Vorrichtung (1) zur lokalen Beeinflussung des Erdmagnetfeldes, wobei an einem flächigen Grundkörper (2) zumindest zwei Fortsätze (4) an beispielsweise einander gegenüberliegenden Kanten (3) angeordnet sind, und die Fortsätze (4) jeweils eine wellenartige Struktur (5) aufweisen und an dem Grundkörper (2) ein das Erdmagnetfeld beeinflussendes Element (6) angeordnet ist.

## Beschreibung

Die Erfindung betrifft eine Vorrichtung zur lokalen Beeinflussung des Erdmagnetfeldes, sowie deren Verwendung zur Anordnung in einem Sitz- und/oder Liegeelement.

Seit Mitte des 19. Jahrhunderts wird das Erdmagnetfeld kontinuierlich in magnetischen Observatorien beobachtet, wobei zur Zeit über 200 Laboratorien weltweit aktiv sind. Hierbei wird die zeitliche Entwicklung des Magnetfeldes in hoher Genauigkeit erfasst. Es wurde beobachtet, dass sich die Stärke des Erdmagnetfeldes seit 1830 um fast 10% verringert hat, in den letzten 100 Jahren allein um etwa 6%. Zur Zeit hat das Magnetfeld der Erde eine Stärke von ca. 30 µT am Äquator, während an den Polen der Betrag etwa doppelt so groß ist. Des weiteren wurde bei der Untersuchung von Sedimentschichten festgestellt, dass etwa alle 4.000 bis 10.000 Jahre eine Umpolung des Erdmagnetfeldes erfolgt. Bei einem derartigen Polsprung wurde in entsprechenden untersuchten Sedimentschichten gehäuft ein Artenwechsel von Kleinorganismen festgestellt, wobei vermutet wird, dass durch die Änderung des Magnetfeldes DNA-Mutationen auftreten. Allgemein ist auch bekannt, dass magnetische bzw. elektromagnetische Felder Einfluss auf das Wohlbefinden von Lebewesen, insbesondere Menschen haben. So gibt es seit Jahren Magnetarmbänder, die das Wohlbefinden steigern; ebenso ist die Magnetfeldtherapie bekannt, bei welcher schmerzhafte Erkrankungen durch die Wirkung eines lokalen Magnetfeldes behandelt werden.

Es ist nun Aufgabe der Erfindung, eine Vorrichtung zu liefern, die die bekannten positiven Auswirkungen von lokalen Magnetfeldern zur Steigerung des Wohlbefindens ausnützt, ohne hierbei einen komplizierten Aufbau aufzuweisen oder aber beispielsweise zusätzliche Energiezufuhr zu benötigen.

Diese Aufgabe wird erfindungsgemäß durch eine Vorrichtung der eingangs erwähnten Art dadurch gelöst, dass an einem flächigen Grundkörper zumindest zwei Fortsätze an beispielsweise zwei einander gegenüberliegenden Kanten angeordnet sind, wobei die Fortsätze jeweils eine wellige Struktur aufweisen und an dem Grundkörper ein das Erdmagnetfeld beeinflussendes Element angeordnet ist. Bei der vorliegenden erfindungsgemäßen Vorrichtung handelt es sich um ein im Wesentlichen flächiges Element, das bevorzugterweise als Rechteck ausgebildet ist, wobei die Fortsätze an zwei einander gegenüberliegenden Kanten angeordnet sind und vorzugsweise geneigt zu der Ebene des Grundkörpers ausgeführt sind. Das an dem Grundkörper angeordnete, das Erdmagnetfeld beeinflussende Element ist beispielsweise ein Permanentmagnet oder aber ein Piezoelement, das bei Druckbelastung ein lokales elektromagnetisches Feld aufbaut. Das das Erdmagnetfeld beeinflussende Element hat die Aufgabe, das zunehmend schwächer werdende Erdmagnetfeld lokal zu verstärken, wobei dieses lokal verstärkte Magnetfeld bevorzugterweise auf bestimmte Körperbereiche des Benutzers wirkt. So wurde festgestellt, dass durch Verstärkung des Magnetfelds im Bereich des Wurzelchakras der Benutzer der erfindungsgemäßen Vorrichtung eine Erhöhung seiner Leistungsfähigkeit feststellte und allgemein über mehr Energie verfügt.

Die Anmelderin hat in einer Pilotstudie festgestellt, dass bei Versuchspersonen, die 15 Minuten auf einem Sitzkissen saßen, in welchem die erfindungsgemäße Vorrichtung angeordnet war, ohne dass die Probanden von dessen Vorhandensein wussten, in einer anschließenden evozierten Elektrophotonen-Gasentladungsuntersuchung (GDV) eine deutliche Verbesserung im Human Energy Field (HEF) auftat, im Gegensatz zu Aufnahmen von Versuchspersonen, die nach dem Sitzen auf einem nicht-präparierten Sitzkissen ohne erfindungsgemäße Vorrichtung aufgenommen wurden.

Das zur Evaluierung der Wirkung der Erfindung eingesetzte Verfahren wurde an der staatlichen Universität St. Petersburg entwickelt (KOROTKOV K.: Human Energy Field Study with GDV Bioelectrography. Backbone Publishing, St. Petersburg, 348 Seiten, 2002; Dobson P. und O'Keefe E.: Int. J. Altern. Complem. Med. 7, 12-17, 2000; Hacker et al., Forsch. Kompl. Med. 12, 315-327, 2005). Dieses Verfahren liefert einen gut reproduzierbaren, physikalisch-biomedizinischen Parameter für Analysen der "relativen Wirksamkeit verschiedener Stressinterventionen" (Korotkov, 2002). Komplementärmedizinische Meridiananalysen von den bei diesem Verfahren erstellten GDV-Coronaprojektionen geben in weiterer Folge auch Hinweise darüber, welche Organsysteme in welcher Art beeinflusst werden. Der Ablauf der GDV-Messungen ist wie folgt zu beschreiben:

Jede Versuchsperson saß für jeweils 15 Minuten auf einem Sitzkissen mit erfindungsgemäßer Vorrichtung, sowie auf einem Sitzkissen ohne lokaler Beeinflussung des Erdmagnetfeldes, wobei weder die Versuchspersonen, noch die die GDV-Messung durchführende Person darüber informiert waren, auf welchem der Sitzkissen die Probanden zu welchem Zeitpunkt saßen. Unmittelbar im Anschluss an die 15minütige Sitzphase wurden GDV-Messungen durchgeführt, wobei die Corona-Entladungen an den zehn Fingerkuppen beider Hände mittels hochsensitiver GDV-Kamera aufgezeichnet und anschließend über eine entsprechende Software ausgewertet wurden. Hierbei hat sich herausgestellt, dass sich der sogenannte GDV-Area-of-Glow-Parameter, der gemäß der Literatur einen Parameter für die Reduktion von Stress darstellt, signifikant erhöhte, wenn die Testperson auf dem mit der erfindungsgemäßen Vorrichtung versehenen Sitzkissen befand. Die Autoren der Studie, Univ. Prof. Dr. Gerhard W. HACKER und HR Prim. Univ.-Prof. Dr. Gernot PAUSER kamen daher zu dem Schluss, dass das Sitzen auf einem Sitzkissen, das mit der erfindungsgemäßen Vorrichtung zur lokalen Beeinflussung des Erdmagnetfeldes ausgestattet ist, signifikante Wirkungen auf den Gesundheitszustand bzw. das Wohlbefinden der Versuchspersonen hat.

In einer ersten Ausführung der Erfindung ist das das Erdmagnetfeld beeinflussende Element ein Permanentmagnet, der bevorzugterweise ein Magnetfeld aufweist, dass im Wesentlichen dreiecksförmige Feldlinienstrukturen aufweist. Auf diese Weise wird ein Multipol-Magnetfeld aufgebaut, das dem natürlichen Erdmagnetfeld nachempfunden ist.

Alternativ hierzu ist in einer weiteren Ausführung der Erfindung das das Erdmagnetfeld beeinflussende Element ein Piezoelement, dass bei Druckbelastung ein lokales elektromagnetisches Feld aufbaut.

In einer bevorzugten Ausführung der Erfindung sind der Grundkörper sowie die beiden Fortsätze aus einem elektrisch leitenden Material, insbesondere aus Federstahl gefertigt. Dieser als Träger für das das Magnetfeld beeinflussende Element fungierende Grundkörper mit den beiden Fortsätzen verstärkt das generierte Magnetfeld ähnlich einer Antenne und folglich auch die Wirkung der erfindungsgemäßen Vorrichtung.

Eine Verstärkung dieser Antennenfunktion wird des weiteren dadurch erzielt, dass das elektrisch leitende Material des Grundkörpers, sowie der Fortsätze zusätzlich magnetisiert ist.

Eine weitere Verbesserung des Wohlbefindens ist mit einer weiteren Ausführung der Vorrichtung zu erzielen, bei welcher die dem das Erdmagnetfeld beeinflussende Element zugewandten Oberfläche des Grundkörpers sowie der Fortsätze mit staubförmigen Partikeln, insbesondere aus Kristall- und/oder Metallstaub beschichtet ist. Es ist allgemein bekannt, dass unterschiedliche Kristalle, wie beispielsweise Bergkristall, Rubine, Diamanten und viele mehr, einen Einfluss auf den energetischen Zustand des menschlichen Körpers haben. Ähnliche Wirkungen sind auch von Metallen wie Gold, Silber, Kupfer usw. bekannt.

Alternativ oder aber auch ergänzend ist in einer weiteren Ausführung der Erfindung vorgesehen, dass in den Tälern der wellenförmigen Struktur der Fortsätze Kristalle angeordnet sind, wobei Kristalle einer Sorte oder aber auch unterschiedliche Kristalle zum Einsatz kommen können.

Die erfindungsgemäße Vorrichtung zur lokalen Beeinflussung des Erdmagnetfeldes ist insbesondere zur Anordnung in einem Sitz- und/oder Liegeelement, insbesondere in Sitzpolster, Matratzen und Sitze von Fahrzeugen und Sportgeräten vorgesehen. Durch die Anordnung der Vorrichtung in Sitz- und/oder Liegeelementen wirkt diese bevorzugt auf das Wurzelchakra, und verstärkt so das Wohlbefinden des Sitzenden oder Liegenden, wobei die Testpersonen häufig berichten, dass sie über mehr Energie und Antriebskraft verfügen.

Im Folgenden wird anhand von nichteinschränkenden Ausführungsbeispielen die Erfindung näher erläutert. Darin zeigen:

| | |
|---|---|
| Fig. 1 | eine Ansicht einer ersten Ausführungsform in einer Ansicht von oben; |
| Fig. 2 | eine Seitenansicht der Vorrichtung aus Fig. 1; |
| Fig. 3 | eine Abbildung des Magnetfeldes des Permanentmagneten aus Fig. 1; und |
| Fig. 4 | eine weitere Variante der Erfindung in einer Ansicht von oben. |

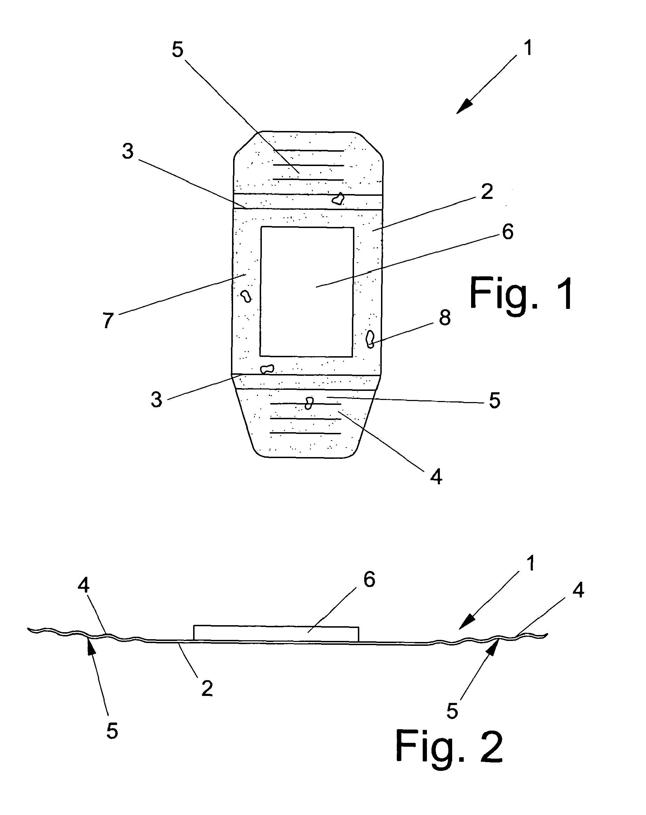

Wie in den Figuren 1 und 2 gezeigt, besteht die erfindungsgemäße Vorrichtung 1 aus einem Grundkörper 2 und zwei an gegenüberliegenden Kanten 3 angeordneten Fortsätzen 4, wobei Grundkörper 2 und Fortsätze 4 in dieser Ausführung einstückig aus einem magnetisierten Federstahl gefertigt sind. Die Fortsätze 4 weisen hierbei eine im wesentlichen sinusförmige Wellenstruktur 5 auf, deren Wellenabstand (Tal zu Tal) zwischen 10 mm und 25 mm und deren Wellenhöhe zwischen 0,5 mm und 3 mm beträgt. Die Wellenstruktur 5 nimmt hierbei direkt Einfluss auf das lokal erzeugte Magnetfeld. Des Weiteren ist die Ebene der Fortsätze 4 leicht geneigt zu der Ebene des Grundkörpers 2 angeordnet.

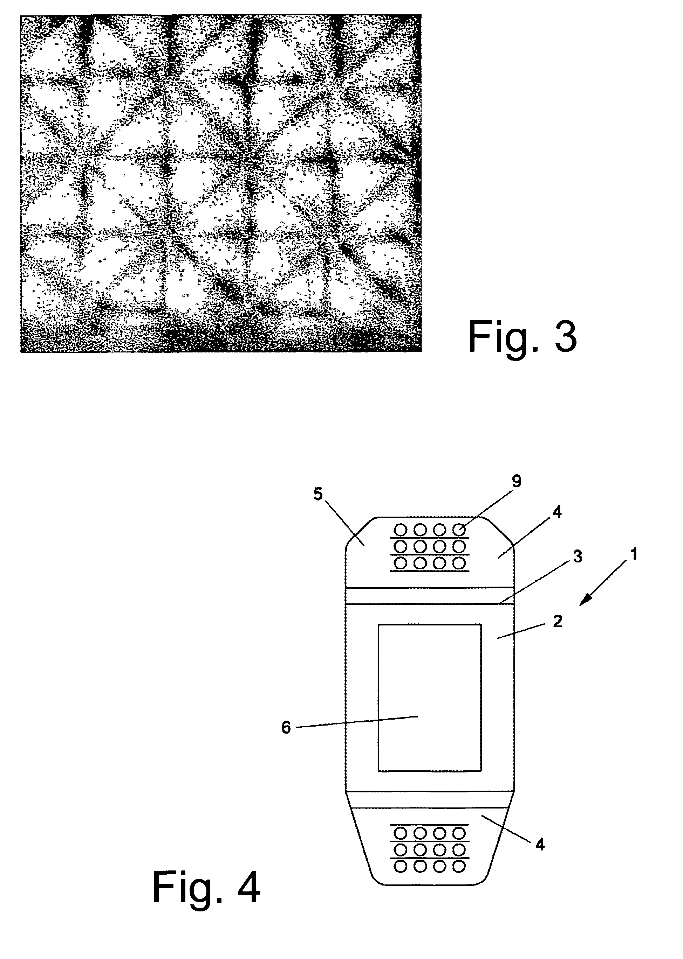

Im Zentrum des Grundkörpers 2 ist ein Permanentmagnet 6 angeordnet, dessen Magnetfeld wie in Fig. 3 dargestellt aufgebaut ist. Die abgebildete Struktur stellt die Feldlinien des Magnetfeldes dar, die eine im wesentlichen dreieckige Gitterstruktur ausbilden.

Die dem Permanentmagneten 6 zugewandte Oberfläche der Vorrichtung 1 ist mit Brillantenstaub 7 sowie Goldpartikel 8 beschichtet.

Bei der in der Figur 4 dargestellten Ausführung der erfindungsgemäßen Vorrichtung 1 sind in den Wellentälern der Fortsätze 4 Kristalle 9, insbesondere Kristalle 9 mit einem hohen Siliziumanteil wie Quarze oder beispielsweise Turmaline mit einem Durchmesser von 0,5 mm bis 2 mm angeordnet.

Die erfindungsgemäße Vorrichtung kann beispielsweise in Sitzpolster von Möbeln oder in Sitze von Fahrzeugen angeordnet sein, wobei sie bevorzugt auf das im Beckenbereich befindliche Wurzelchakra einwirkt. Ihre positive Beeinflussung des allgemeinen Wohlbefindens als auch ihre belebende Wirkung macht ihre Anwendung insbesondere im Sport durch den Einbau in Sätteln von beispielsweise Fahrrädern oder auch zum Beispiel in Rückenprotektoren interessant. Bei der Verwendung in Matratzen hat sich gezeigt, dass hier eine Anordnung der erfindungsgemäßen Vorrichtung im Fußbereich vorteilhaft ist, weil sie an dieser Stelle beruhigend und weniger belebend wirkt.

## Patentansprüche

1. Vorrichtung (1) zur lokalen Beeinflussung des Erdmagnetfeldes, **dadurch gekennzeichnet, dass** an einem flächigen Grundkörper (2) zumindest zwei Fortsätze (4) an einander gegenüberliegenden Kanten (3) angeordnet sind, wobei die Fortsätze (4) jeweils eine wellenartige Struktur (5) aufweisen und an dem Grundkörper (2) ein das Erdmagnetfeld beeinflussendes Element (6) angeordnet ist.

2. Vorrichtung (1) nach Anspruch 1, **dadurch gekennzeichnet, dass** das das Erdmagnetfeld beeinflussende Element (6) ein Permanentmagnet ist.

3. Vorrichtung (1) nach Anspruch 2, **dadurch gekennzeichnet, dass** der Permanentmagnet ein Magnetfeld aufweist, das im Wesentlichen dreiecksförmige Feldlinienstrukturen aufweist.

4. Vorrichtung (1) nach Anspruch 1, **dadurch gekennzeichnet, dass** das das Erdmagnetfeld beeinflussende Element (6) ein Piezoelement ist, das bei Druckbelastung ein lokales elektromagnetisches Feld aufbaut.

5. Vorrichtung (1) nach einem der Ansprüche 1 bis 4, **dadurch gekennzeichnet, dass** der Grundkörper (2) sowie die beiden Fortsätze (4) aus einem elektrisch leitenden Material, insbesondere aus Federstahl gefertigt sind.

6. Vorrichtung (1) nach Anspruch 5, **dadurch gekennzeichnet, dass** das elektrisch leitende Material des Grundkörpers (2) sowie der Fortsätze (4) zusätzlich magnetisiert ist.

7. Vorrichtung (1) nach einem der Ansprüche 1 bis 6, **dadurch gekennzeichnet, dass** die dem das Erdmagnetfeld beeinflussende Element (6) zugewandte Oberfläche mit staubförmigen Partikeln (7, 8) beschichtet ist.

8. Vorrichtung (1) nach Anspruch 7, **dadurch gekennzeichnet, dass** die staubförmigen Partikel (7, 8) aus Kristall- und/oder Metallstaub hergestellt sind.

9. Vorrichtung (1) nach einem der Ansprüche 1 bis 8, **dadurch gekennzeichnet, dass** in den Tälern der wellenförmigen Struktur (5) der Fortsätze (4) Kristalle (9) angeordnet sind.

10. Verwendung der Vorrichtung (1) zur lokalen Beeinflussung des Erdmagnetfeldes nach einem der Ansprüche 1 bis 9 zur Anordnung in einem Sitzund/oder Liegeelement, insbesondere in Sitzpolster, Matratzen und Sitze von Fahrzeugen und Sportgeräten.
